# EUROPEAN PATENT SPECIFICATION

(11) **EP 3 167 881 B1**
(45) Date of publication and mention of the grant of the patent: **28.08.2019**
(21) Application number: 15380045.3
(22) Date of filing: 19.10.2015
(51) Int. Cl.: A61K 31/185, A61K 31/205, A61P 19/00, A61P 19/04, A61P 19/02, A61P 21/00, A61P 27/16

(54) **LOCALLY ADMINISTERED ETHAMSYLATE AS A MEDICAMENT**
LOKAL VERABREICHTES ETHAMSYLATE ALS MEDIKAMENT
ETHASMSYLATE ADMINISTRÉ LOCALEMENT COMME MÉDICAMENT

(43) Date of publication of application: 17.05.2017
(73) Proprietor: DOBECURE, S.L., 28017 Madrid Madrid (ES)
(72) Inventor: CUEVAS, SANCHEZ, Pedro, 28035, Madrid (ES); GIMENEZ GALLEGO, Guillermo, 28049 Madrid (ES); FERNANDEZ JAEN, Tomás, 28660, Boadilla del Monte (ES)
(74) Representative: Monzón de la Flor, Luis Miguel

(56) References cited:
- WO-A1-2008/020042
- JOON MOON ET AL: "Role of Angiogenic Factors in Airway Remodeling in an Allergic Rhinitis Murine Model", ALLERGY, ASTHMA & IMMUNOLOGY RESEARCH, vol. 4, no. 1, 1 January 2012 (2012-01-01) , page 37, XP055268447, ISSN: 2092-7355, DOI: 10.4168/aair.2012.4.1.37
- NYALL R. LONDON ET AL: "The role of vascular endothelial growth factor and vascular stability in diseases of the ear", THE LARYNGOSCOPE, vol. 124, no. 8, 28 August 2014 (2014-08-28), pages E340-E346, XP055268449, United States ISSN: 0023-852X, DOI: 10.1002/lary.24564
- CUEVAS P ET AL: "Treatment of dry age-related macular degeneration with dobesilate", BMJ CASE REPORTS, BMJ PUBL. GROUP, UK, vol. 2012, 22 June 2012 (2012-06-22), pages 1-4, XP009177558, ISSN: 1757-790X, DOI: 10.1136/BCR.02.2012.5942
- P. CUEVAS ET AL: "Dramatic response to inhaled dobesilate in a patient with lung squamous cell cancer", BMJ CASE REPORTS, vol. 2012, no. sep05 1, 5 September 2012 (2012-09-05), XP055268647, UK ISSN: 1757-790X, DOI: 10.1136/bcr-2012-006622

## Description

### Field of the Invention

The invention describes the use of etamsylate administered locally in patients with otitis or rhinitis.

### Background of the invention

Rhinitis is a very common disease and represents a health problem for both children and adult globally. Rhinitis can be allergic or occur without any IgE-mediated sensitization to aeroallergens. Common symptoms include nasal congestion, itching, rhinorhea and sneezing. Antihistamines and glucocorticoids arc often the first line treatments administered for rhinitis. However, these drugs were associated with side effects (Sadaba B et al. Ther Clin Risk Manag 2013; 9: 197-205; Yurttas V et al. J Photochem Photobiol 2015; 149: 289-291)

External otitis is an inflammatory process of the external auditory canal. This condition was found to be disabling enough to cause 36% of patients to interrupt their daily activities for a median duration of four days with 21% bed rest (van Asperen IA et al. BMJ 1995: 311: 1407-1410). The two most characteristic symptoms of external otitis are otalgia (ear discomfort) and otorrhea. The ear discomfort can range from pruritus to severe pain that is exacerbated by motion of the ear, including chewing. If inflammation causes sufficient swelling to occlude the external auditory canal, the patient may also complain of aural fullness and loss of hearing. The ear drops steroids may decrease the inflammation and oedema of the auditory canal and resolve symptoms but not all studies have shown a benefit. In addition, a topical steroid can be a topical sensitizer. Furthermore, fungal external otitis (otomycosis) may be aggravated by the use of steroid-containing drops.

Furthermore, novel and safe treatment options for all these diseases are needed and must be considered unmet medical need. It may be possible to achieve faster symptoms relief through direct delivery of a medication to the pathologic tissue.

Etamsylate (Dicynone®. Sanofi-Aventis. Paris.Fraoce) is a safe synthetic haemostatic agent that has been used orally and systematically for many years. However, local delivery seems a better choice in order to reach appropriate therapeutic concentrations of etamsylate in the case of well delimited targets, as is the case of external otitis and rhinitis.

Inventors have surprisingly found that local administration of etamsylate (administered as a 12.5% solution of diethylamonium 2,5-dihydroxy benzenesulfonate (etamsylate. Dicynone®, Sanofi-Aventis. Paris France) shows clinical benefits in patients with external otitis and seasonal allergic rhinitis.

After approval of our Institutional Ethical Committee, patients signed an informed consent form, which includes a comprehensive description of the proposed procedures.

### Detailed description of the invention

As used throughout the disclosure, the following terms, unless otherwise indicated, shall be understood to have the following meanings.

"Patient" refers to animals, preferably mammals, most preferably humans, and includes males and females.

"Effective amount" refers to the amount of the compound and/or composition that is effective to achieve its intended purpose.

"Treating"; "treat" or "treatment" refers to using the compound or compositions of the present invention either prophylactically to prevent the symptoms of the disease or disorder, or therapeutically to ameliorate an existing condition.

"Therapeutic agent" includes any therapeutic agent which can be used to treat or prevent the disease described herein. Therapeutic agents include, but are not limited to, antioxidants, anti-inflammatory compounds and the like. Therapeutic agent includes the pharmaceutically acceptable salts thereof, pro-drugs and pharmaceutical derivatives thereof.

The compound and compositions of the invention can be administered by any available and effective delivery system, including, but not limited to by local application, or by intraarticular administration in dosage unit formulations containing conventional non-toxic pharmaceutical acceptable carriers, adjuvant and vehicles as desired.

Local administration of the compound and compositions invention can be in the form of creams, gels, ointments, dispersions, solid sticks, emulsions, and microemulsions that can be formulated according to the conventional methods using suitable excipicnts. Injectable preparations, for example, sterile injectable aqueous suspensions can be formulated according to the known act using suitable dispersing agents, wetting agents and/or suspending agents. The sterile injectable preparations can also be a sterile injectable solution or suspension in a non-toxic acceptable diluent or solvent. Among the acceptable vehicles and solvents that can be used are water, Ringer's solution, and isotonic sodium chloride solution.

Generally, the dosage required providing an effective amount of the compound and composition, which can be adjusted by one of ordinary skill in the art, will vary depending on the extent of the disease and frequency of treatment.

The subject treated with etamsylate is an animal, preferably a mammal and more specifically a human. The main object of the present invention is directed to the treatment or prevention of the diseases described herein, mainly in humans.

The duration of treatment will typically depend on the particular condition, its severity, the condition of the patient, and the like, and will readily be determined by one of skill in the art. Illustrative courses of therapy include 1 week, 2 weeks, 3 weeks, 4 weeks, 5 weeks, 6 weeks, 7 weeks, 8 weeks, 9 weeks, 10 weeks, 11 weeks, 12 weeks, 3.5 months, 4 months, 4.5 months, 5 months, 6 months, 9 months, a year or longer as needed.

The term "pharmacuetically acceptable" refers to physiologically tolerable molecular entities and compositions which do not typically produce an adverse reaction when administered to a human or veterinary patient. Preferably, as used herein, the term "pharmaceutically acceptable" means that it is approved by a regulatory agent of the Federal or a State Government or listed in the U.S. Pharmacopoeia or other generally recognized pharmacopoeia as suitable for use In mammals, and more particularly in humans,

The compound used in the invention may be used at a level from about 1mg/kg of weight to about 200mg/kg of weight without evidencing toxicity.

### Examples

The following non limiting examples describe and permit to one of ordinary skill in the art to use the present invention.

### Example 1.- Effect of etamsylate ear drops in acute external otitis

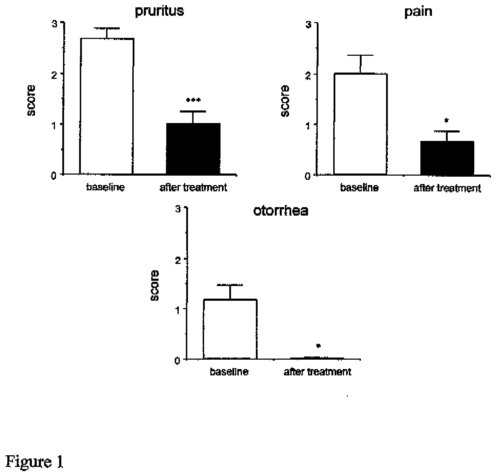

Six patients of both sexes with acute external otitis participated in this study. Patients were asked before and during treatment the otitis symptoms experienced. Otitis symptoms (pain, pruritus, hypoacusia and otorrhea) were rated as; 0: none, 1: mild, 2: moderate and 3 severe. Patients applied etamsylate (5 ear drops twice a day) for one week in the affected auditory canal. Mean clinical symptoms scores before and after one week of treatment with etamsylate were compared by paired *t*-test. being p<0.05 considered significant. Patients reported that all symptoms improved significantly after treatment (Fig. 1). Patients did not experience any adverse effects related to etamsylate treatment.

### Example 2.- Effect of etamsylate spray in seasonal allergic rhinitis

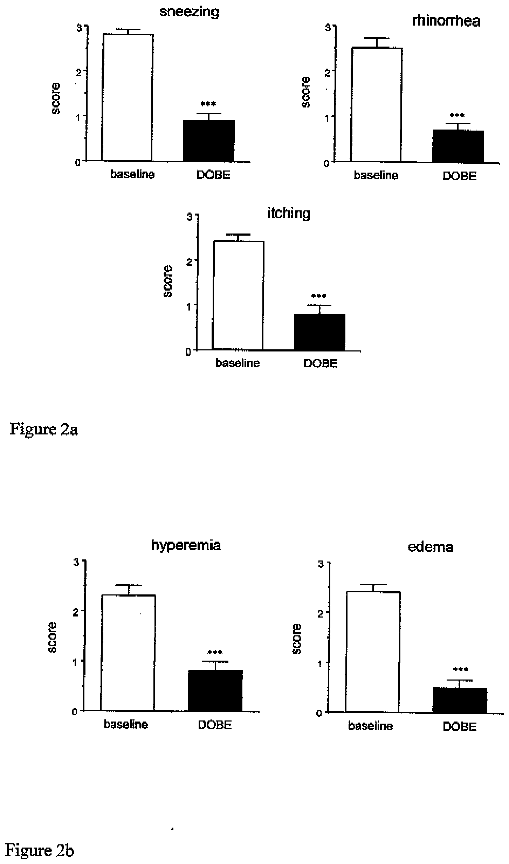

Seven patients with seasonal allergic rhinitis of both sexes and age ranging between 30 to 65 years participated in this study. Patients were asked before and daring treatment to rate the rhinitis symptoms experienced. The Total Nasal Symptoms Score (TNSS) comprises the following symptoms of rhinitis: sneezing, rinorrhea and itching and rates each individual symptoms on a 4-points scale (0 none, 1: mild, 2: moderate and 3: severe). Mucosal allergic rhinitis signs such as hyperemia and edema were evaluated by rhinoscopy at baseline and after treatment. Clinical signs were graded using the following scale: 0: no signs of involvement, 1: moderate and 2-3: severe. Patients applied etamsylate spray (3 times/day) to each nostril for one week. Mean clinical scores before and after one week of treatment with etamsylate were compared by paired *t*-test. p < 0.05 was considered significant. Patients reported that all symptoms improved after the first day of treatment both in the morning and at night. After treatment all symptoms scores were significantly reduced (Fig. 2). Patients did not experience any side-effect related to etamsylate treatment.

### Figures legends

**Figure 1****.- Effect of etamsylate ear drops in acute external otitis**
   Effect of one week of treatment with etamsylate on otitis symptoms. Ear drops application of etamsylate improved symptoms in patients. Data from the 6 patients are expressed as mean±SEM. * indicates p<0.05 and *** p < 0.001 vs. baseline by paired *t-*test.
**Figure 2****.- Effect of etamsylate spray in seasonal allergic rhinitis**
   Improvement of all symptoms (a) and signs (b) of seasonal allergic rhinitis by nostril spray administration of etamsylate. Data collected in seven patients before and seven days after treatment. Results were compared by paired t-test. ** indicates p<0,01 vs baseline.

## Claims

1. Etamsylate for use in the treatment of otitis and rhinitis, wherein etamsylate is locally applied.

2. Etamsylate for use according to claim 1 wherein the treatment is to be carried out in humans or mammals

3. Etamsylate for use according to claims 1 and 2 wherein etamsylate further contains one or more pharmaceutically acceptable excipients or active compounds.

## Patentansprüche

1. Etamsylat zur Verwendung bei der Behandlung von Otitis und Rhinitis, wobei Etamsylat lokal angewendet wird.

2. Etamsylat zur Verwendung nach Anspruch 1, wobei die Behandlung an Menschen oder Säugetieren erfolgt.

3. Etamsylat zur Verwendung nach Ansprüchen 1 und 2, wobei Etamsylat darüber hinaus ein oder mehrere pharmazeutisch zugelassene Arzneimittelträger oder aktive Wirkstoffe enthält.

## Revendications

1. Etamsylate destiné à être utilisé dans le traitement de l'otite et de la rhinite, dans lequel l'etamsylate est appliqué localement.

2. Etamsylate destiné à être utilisé selon la revendication 1, dans lequel le traitement doit être effectué chez les humains ou des mammifères.

3. Etamsylate destiné à être utilisé selon les revendications 1 et 2, dans lequel l'etamsylate contient en outre un ou plusieurs excipients ou composés actifs pharmaceutiquement acceptables.
